# EUROPEAN PATENT APPLICATION

(11) **EP 4 005 633 A2**
(43) Date of publication of application: **01.06.2022**
(21) Application number: 22155571.7
(22) Date of filing: 08.02.2022
(51) Int. Cl.: A61P 3/06, A61P 9/00, C07D 239/42, A61K 31/505

(54) **CRYSTALLINE FORM OF ROSUVASTATIN ALLYL ESTER**

(71) Applicant: Sawant, Mohit Manikrao, 411043 Pune (IN)
(72) Inventor: GABRSCEK, Miran, 4220 Skofja Loka (SI)
(74) Representative: Prüfer & Partner mbB Patentanwälte · Rechtsanwälte

(57) **Abstract**

The present invention relates to crystalline rosuvastatin allyl ester (1), to a process for its manufacture, to pharmaceutical compositions comprising it as well as the use of crystalline (1) in medicine.

## Description

The present invention relates to a crystalline rosuvastatin allyl ester (**1**), and its use in medicine, in particular as a prodrug of rosuvastatin, a pharmaceutical agent useful, for example, in the treatment of hyperlipidaemia, hypercholesterolemia and atherosclerosis, as well as other diseases or conditions in which HMG CoA reductase is implicated. The invention also relates to a process for the manufacture of the crystalline (**1**), pharmaceutical compositions comprising it as well as the use of said pharmaceutical compositions in medicine.

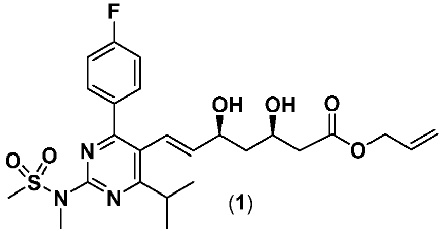

### Background of invention

As generally known, any active principle may exist under amorphous or different crystalline forms (polymorphs), either as pure compound or in forms in which, in the structure of the crystal, are present molecules of water (hydrates) or of another solvent (solvates); besides, in case of hydrates and solvates, the ratio between the number of molecules of active principle and molecules of water or solvent may vary, giving rise to different solid forms of the compound. Different salts and solid-state forms of an active pharmaceutical ingredient may possess different properties which may provide opportunities to modify the properties and characteristics of a solid active pharmaceutical ingredient.

Differences in physicochemical properties of solid forms can play a crucial role for the improvement of pharmaceutical compositions, for example, pharmaceutical formulations with improved dissolution profile or with improved stability or shelf-life can become accessible due to an improved solid-state form of an active pharmaceutical ingredient. Also processing or handling of the active pharmaceutical ingredient during the formulation process may be improved. New solid-state forms of an active pharmaceutical ingredient can thus have desirable processing properties. They can be easier to handle, better suited for storage, and/or allow for better purification.

Also, the tendency of a drug substance to absorb water from the environment can negatively affect the pharmaceutical behaviour and quality of a drug product. Water absorption for example can lead to chemical degradation of the physical form (e.g., via hydrate formation), changes in dissolution behaviour and influence powder properties such as flowability, compactability, tableting and compression behaviour etc. Furthermore, the sudden appearance or disappearance of a metastable polymorph can present a problem in process development. Similarly, serious pharmaceutical consequences arise if solid-state transformations occur in a dosage form.

For these reasons, chemical compounds useful in the pharmaceutical field are systematically screened looking for the physical form(s) that present an improved set of production, storage and/or handling properties, and which result in an improved administration to the patients.

The International patent application WO 2011/160974 A1 discloses an unspecified form of (**1**) prepared by dissolving it in ethyl acetate, washing with water and concentrating the organic layer under reduced pressure.

The International patent application, WO 2019/139919 A1, discloses the preparation of (**1**) in the form of a light yellow oil after purification by column chromatography.

R&D Disclosure RD689019 provides a solubility prediction of (**1**) in a series of solvent.

However, no information is provided in any of these documents about any useful properties from the standpoint of the pharmaceutical industry, neither regarding ease of handling of the resulting (**1**) in the production of formulations, nor regarding the stability of (**1**) when prepared in one of these forms.

There is thus a need to provide a solid-state form of rosuvastatin allyl ester (**1**) which possesses physicochemical properties allowing the reliable production of a safe and efficacious drug product comprising it. In particular, there is a need for a solid-state form of (**1**) which is diasteromerically and/or enantiomerically pure, and/or is stable upon storage of the active pharmaceutical ingredient (API), during formulation and throughout the whole shelf-life of the drug product comprising it.

### Summary of the Invention

The present invention solves one or more of the above mentioned problems by providing rosuvastatin allyl ester (**1**) in crystalline form which possesses favourable physicochemical properties for a drug substance intended for use in an oral solid dosage form. Said properties concern for example chemical stability, physical stability, hygroscopicity, solubility, dissolution, morphology, crystallinity, flowability, compactability and wettability.

The crystalline form of (**1**) according to the present invention possesses physical properties allowing the reliable production of a safe and efficacious pharmaceutical drug product, e.g., a solid dosage form such as a capsule or a tablet comprising rosuvastatin allyl ester (**1**). In particular, the crystalline rosuvastatin allyl ester (1**)** of the present invention is polymorphically stable and preserve its crystal structures during manufacture and storage of a solid dosage form comprising it. For example, the crystalline form of the present invention, is thermally stable against temperature stress, e.g., it does not show any thermal events in the DSC curve up to a temperature of about 90°C when measured at a heating rate of 10 K/min. Moreover, crystalline (**1**), being anhydrous and/or non-solvated, is stable against temperature and moisture stress. In addition, it is non-hygroscopic and preserve its crystal structure regardless relative humidity of the environment.

### Definitions

All terms used in this application, unless otherwise specified, are to be understood in their ordinary meaning as known in the technical field. Other more specific definitions of certain terms used in this application are listed below and are intended to be applied uniformly to the entire application, unless indicated otherwise.

The term *"measured at a temperature in the range of from 20 to 35 °C*" refers to a measurement under standard conditions. Typically, standard conditions mean at room temperature, i.e. a temperature in the range of from 20 °C to 35 °C. Standard conditions preferably means a temperature from 22 °C to 30 °C, more preferably from 25 °C to 28 °C. Even more preferably, standard conditions imply a relative humidity of 20-70%, for example of 30-60%.

The symbol 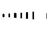 (dashed bond), when present in some of the formulae of the description and/or the claims, indicates that the substituent is directed below the plane of the sheet.

The symbol 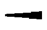 (wedge bond), when present in some of the formulae of the description and/or the claims, indicates that the substituent is directed above the plane of the sheet.

The term *"reflection"* with regard to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to common general knowledge, long-range order normally extends over approximately 100 to 1000 atoms, whereas short-range order is over a few atoms only.

The term *"essentially the* same" with reference to powder X-ray diffraction means that variabilities in reflection positions and relative intensities of the reflections are to be taken into account. For example, a typical precision of the 2-Theta (2Θ) values is in the range of ± 0.2° 2Θ, preferably in the range of ± 0.1° 2Θ, more preferably in the range of ± 0.05° 2Θ. Thus, a reflection that appears at 15.3° 2Θ can appear from 15.1° to 15.5° 2Θ, preferably from 15.2° to 15.4° 2Θ, more preferably from 15.25° to 15.35° 2Θ, on most X-ray diffractometers under standard conditions. Furthermore, one skilled in the art will appreciate that relative reflection intensities will show inter-apparatus variability as well as variability due to degree of crystallinity, preferred orientation phenomena, sample preparation and other factors known to those skilled in the art.

The term *"solid-state form"* as used herein refers to any crystalline and/or amorphous phase of a compound. Crystalline phases include anhydrous, hydrate, solvate, or non-solvated forms of a given compound, its salts and co-crystals, as well as all the polymorphs of any one of them. The term *"anhydrous"* as used herein refers to a crystalline solid where no water is cooperated in or accommodated by the crystal structure. Anhydrous forms may still contain residual water, which is not part of the crystal structure but may be adsorbed on the surface or absorbed in disordered regions of the crystal. Typically, an anhydrous form does not contain more than 1.0% by weight, preferably not more than 0.5% by weight, based on the weight of the crystalline form. The water content can be determined by Karl-Fischer Coulometry and/or by thermogravimetric analysis (TGA), e.g., by determining the mass loss in the range of from 30 to 180°C at a heating rate of 10 K/min.

The term *"non-solvated'* as used herein indicates that no organic solvent is cooperated in or accommodated by the crystal structure. Non-solvated forms may still contain residual organic solvents, which are not part of the crystal structure but may be adsorbed on the surface or absorbed in disordered regions of the crystal. Typically, a non-solvated form does not contain more than 1.0 weight%, preferably not more than 0.5 weight% of organic solvents, based on the weight of the crystalline form. The organic solvent content can be determined by thermogravimetric analysis (TGA), e.g., by determining the mass loss in the range of from 30 to 180°C at a heating rate of 10 K/min or by ¹H-NMR.

Rosuvastatin allyl ester (1) has more than one stereogenic center. Accordingly, it may exist and may be used or isolated in enantiomerically pure forms, as enantiomeric enriched mixtures as well as in diasteromerically pure forms or as diastereomeric enriched mixtures. It is to be understood that the process of the present invention can give rise to any of the previous forms or mixtures or a combination thereof. It is to be further understood that the products of the process described herein, can be isolated as enantiomerically and/or diasteromerically pure forms or as enantiomerically and/or diasteromerically enriched mixtures.

A mixture of (R,S)-enantiomers can contain the two enantiomers in any ratio to each other. The enantiomeric purity is generally expressed as "enantiomeric excess" or ee and is defined, for example for the (S) enantiomer, as [(*S-R*)/(*R+S*)] X 100, wherein *S* and *R* are respectively the amounts of the (S) and (R) enantiomers (as determined for example by GC or HPLC on a chiral stationary phase or polarimetry).

The term *"racemic"* refers to a sample of a chiral compound which contains both the (+) and (-) isomers in equal amount.

The term *"enantiomerically enriched"* as used herein means that one of the enantiomers of rosuvastatin allyl ester (1) is present in excess compared to the other enantiomer.

The term "enantiomerically pure" as used herein means that the enantiomeric purity of rosuvastatin allyl ester (1) is usually at least about 95%, preferably at least 98%, more preferably at least 99%, even more preferably at least 99.5%.

The term *"diasteromerically enriched"* as used herein means that, for example, the (3R,5S)-isomer of rosuvastatin allyl ester (1) is present in excess compared to the other optical isomers, i.e. (3R,5R)-isomer (the enantiomer) and/or (3S,5S)-isomer and (3S,5R)-isomer (the diastereomers).

The term *"diasteromerically* pure" as used herein means that the diastereomeric purity of rosuvastatin allyl ester (1) is usually at least about 95%, preferably at least 98%, more preferably at least 99%, even more preferably at least 99.5%.

A stereomerically pure composition of (1), preferably the (3R,5S)-isomer of rosuvastatin allyl ester (1), will be substantially free of other optical isomers of the same compound. A typical stereomerically pure composition normally comprises more than about 95% by weight of (3R,5S)-isomer of (1) and less than about 5% by weight of other optical isomers of the same compound, preferably more than about 98% by weight of (3R,5S)-isomer of (1) and less than about 2% by weight of the other optical isomers, more preferably more than about 99% by weight of (3R,5S)-isomer of (1) and less than about 1% by weight of the other optical isomers, and even more preferably more than about 99.5% by weight of (3R,5S)-isomer of (1) and less than about 0.5% by weight of the other optical isomers of the same compound.

As used herein, the term *"about'* means within a statistically meaningful range of a value. Such a range can be within an order of magnitude, typically within 10%, more typically within 5%, even more typically within 1% and most typically within 0.5% of the indicated value or range. Sometimes, such a range can lie within the experimental error, typical of standard methods used for the measurement and/or determination of a given value or range.

The term *"mass"* defines the combination of substrates, reagents, solvents, and products on which a physical or chemical transformation is carried out.

The term *"seed"* refers to a crystalline substance that is added to a dispersion of the same substance to induce its crystallization. Seeding with a specific crystalline form has often the useful effect of promoting crystallization of the substance in the same crystalline form of the seed. Crystalline rosuvastatin allyl ester (1) according to the present invention may be characterized by a powder X-ray diffractogram "as *shown in"* a figure. The person skilled in the art understands that factors such as variations in instrument type, response and variations in sample directionality, sample concentration, sample purity, sample history and sample preparation may lead to variations, for example relating to the exact reflection or peak positions and intensities. However, a comparison of the graphical data in the figures herein with the graphical data generated for an unknown physical form and the confirmation that two sets of graphical data relate to the same crystal form is well within the knowledge of a person skilled in the art.

A *"prodrug"* is a compound that is generally not pharmacologically active. However, when activated, typically *in vivo* by enzymatic or hydrolytic cleavage to convert the prodrug to a drug, the administration of the prodrug to the individual will have had the intended medical effect. Prodrugs are typically formed by chemical modification of a biologically active compound. One purpose of employing a prodrug, for oral administration, for example, is to increase intestinal or site-specific absorption. Another purpose is to reduce local side effects, such as gastrointestinal irritation.

As used herein, the term *"effective amount"* in conjunction with the crystalline rosuvastatin allyl ester (1) of the present invention encompasses an amount of the crystalline (1) which causes the desired therapeutic or prophylactic effect.

The term *"pharmaceutically acceptable excipient*" as used herein refers to substances, which do not show a significant pharmacological activity at the given dose and that are optionally included in a pharmaceutical composition in addition to the active pharmaceutical ingredient. Excipients may take the function of vehicle, diluent, release agent, disintegrating agent, dissolution modifying agent, absorption enhancer, stabilizer or a manufacturing aid among others. Excipients may include fillers (diluents), binders, disintegrants, lubricants and glidants.

The terms *"filler"* or *"diluent"* as used herein refer to substances that are used to dilute the active pharmaceutical ingredient prior to delivery. Diluents and fillers can also serve as stabilizers.

As used herein the term *"binder"* refers to substances which bind the active pharmaceutical ingredient and pharmaceutically acceptable excipient together to maintain cohesive and discrete portions.

The term *"disintegrant"* or *"disintegrating agent"* as used herein refers to substances which, upon addition to a solid pharmaceutical composition, facilitate its break-up or disintegration after administration and permits the release of the active pharmaceutical ingredient as efficiently as possible to allow its rapid dissolution.

The term *"lubricant"* as used herein refers to substances which are added to a powder blend to prevent the compacted powder mass from sticking to the equipment during tableting or encapsulation process. They help the ejection of the tablet from the dies and can improve powder flow.

The term *"glidanf'* as used herein refers to substances which are used for tablet and capsule formulations in order to improve flow properties during tablet compression and to produce an anti-caking effect.

### Brief Description of the Drawings

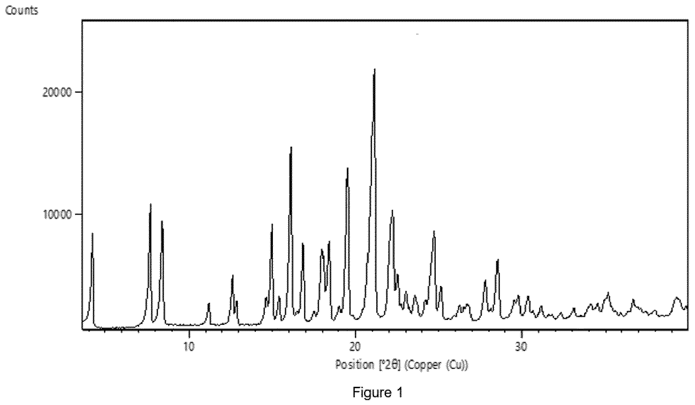

**FIG. 1****.** illustrates a representative PXRD of crystalline rosuvastatin allyl ester (**1**) according to the present invention. The x-axis shows the scattering angle in °2Θ, the y-axis shows the intensity of the scattered X-ray beam in counts of detected photons.

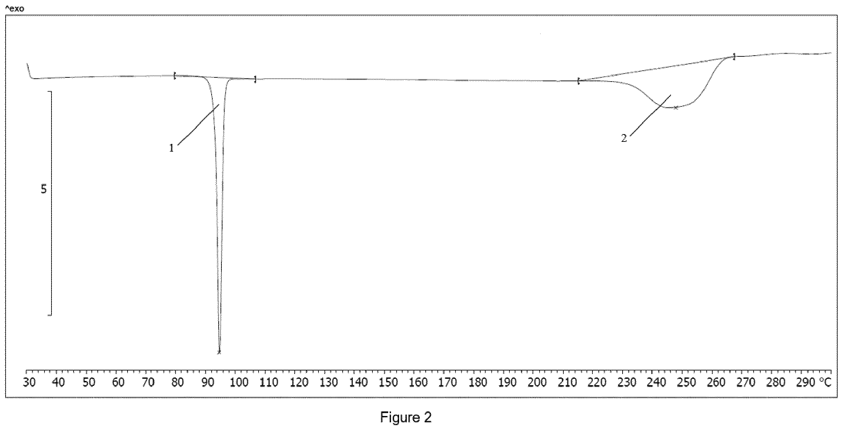

**FIG. 2****.** illustrates a representative DSC curve crystalline rosuvastatin allyl ester (**1**) according to the present invention, wherein the data corresponding to the two areas are included below: (1) Integral -171.71 mJ, normalized -78.77 Jg⁻¹, Onset 93.00 °C, Peak 94.16 °C and (2) Integral -290.61 mJ, normalized -133.31 Jg⁻¹, Onset 229.78 °C, Peak 247.73 °C. The x-axis shows the temperature in degree Celsius (°C), the y-axis shows the heat flow rate in Watt per gram (W/g) with endothermic peaks going down.

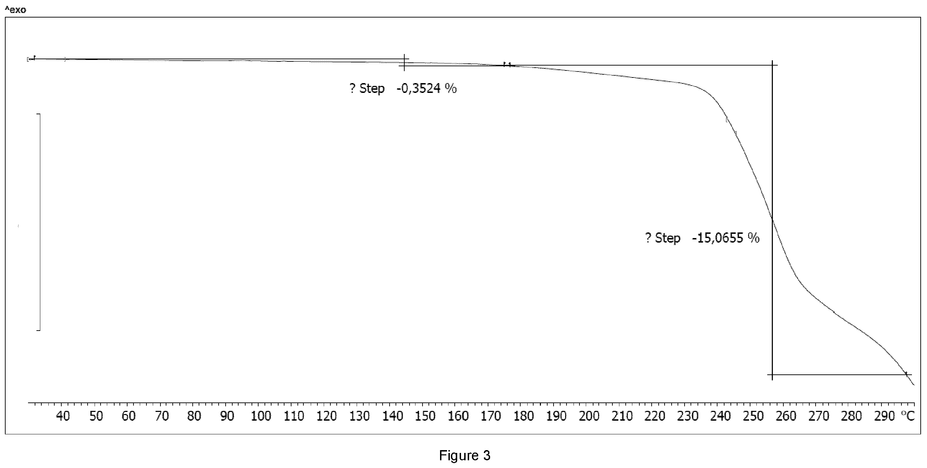

**FIG. 3****.** illustrates a representative TGA curve of crystalline rosuvastatin allyl ester (**1**) of the present invention, which shows a loss of 0.35% in the area from 30 °C to 180 °C. The x-axis shows the temperature in degree Celsius (°C), the y-axis shows the mass (loss) of the sample in weight percent (w-%).

### Detailed Description of the Invention

In a first aspect the present invention relates to a solid-state form of rosuvastatin allyl ester (**1**) that is crystalline, as determined by one of the analytical methods known in the field for characterizing crystalline materials, such as, for example, powder X-ray diffraction, DSC and/or TGA, preferably powder X-ray diffraction.

Preferably the crystalline rosuvastatin allyl ester (1**)** - for brevity referred to in the rest of the description and in the claims as form α (alpha) - is anhydrous and/or non-solvated and/or non-hygroscopic.

Said form α can be further characterized by any one of the following features (i) to (xi):
(i) an X-ray powder diffraction pattern that, when collected with the Kα radiation of copper (λ = 1.5419 Å) and a temperature from 20 to 30°C, shows main reflections at 16.14° (± 0.20° 2Θ) and 21.13° (± 0.20° 2Θ); and/or
(ii) an X-ray powder diffraction pattern that, when collected with the Kα radiation of copper (λ = 1.5419 Å) and a temperature from 20 to 30°C, shows main reflections at 7.70° (± 0.20° 2Θ), 16.14° (± 0.20° 2Θ), 19.53° (± 0.20° 2Θ) and 21.13° (± 0.20° 2Θ); and/or
(iii) an X-ray powder diffraction pattern that, when collected with the Kα radiation of copper (λ = 1.5419 Å) and a temperature from 20 to 30°C, shows main reflections at 7.70° (± 0.20° 2Θ), 8.41° (± 0.20° 2Θ), 14.99° (± 0.20° 2Θ), 16.14° (± 0.20° 2Θ), 19.53° (± 0.20° 2Θ), 21.13° (± 0.20° 2Θ) and 22.24° (± 0.20° 2Θ); and/or
(iv) an X-ray powder diffraction pattern that, when collected with the Kα radiation of copper (λ = 1.5419 Å) and a temperature from 20 to 30°C, shows main reflections at 7.70° (± 0.20° 2Θ), 8.41° (± 0.20° 2Θ), 14.99° (± 0.20° 2Θ), 16.14° (± 0.20° 2Θ), 16.87° (± 0.20° 2Θ), 18.44° (± 0.20° 2Θ), 19.53° (± 0.20° 2Θ), 21.13° (± 0.20° 2Θ), 22.24° (± 0.20° 2Θ) and 24.74° (± 0.20° 2Θ); and/or
(v) an X-ray powder diffraction pattern that, when collected with the Kα radiation of copper (λ = 1.5419 Å) and a temperature from 20 to 30°C, shows main reflections at 7.70° (± 0.20° 2Θ), 8.41° (± 0.20° 2Θ), 12.63° (± 0.20° 2Θ), 14.99° (± 0.20° 2Θ), 16.14° (± 0.20° 2Θ), 16.87° (± 0.20° 2Θ), 17.96° (± 0.20° 2Θ), 18.44° (± 0.20° 2Θ), 19.53° (± 0.20° 2Θ), 21.13° (± 0.20° 2Θ), 22.24° (± 0.20° 2Θ), 24.74° (± 0.20° 2Θ) and 28.55° (± 0.20° 2Θ); and/or
(vi) an X-ray powder diffraction pattern that, when collected with the Kα radiation of copper (λ = 1.5419 Å) and a temperature from 20 to 30°C, is essentially as shown in Figure 1; and/or
(vii) a DSC trace comprising an endothermic peak having an onset at a temperature of (93 ± 5)°C, preferably of (93 ± 3)°C, more preferably of (93 ± 2)°C, even more preferably of (93 ± 1)°C, when measured at a heating rate of 10 K/min; and/or
(viii) a DSC trace that, when measured at a heating rate of 10 K/min, is essentially as shown in Figure 2; and/or
(ix) a TGA curve showing a mass loss of not more than 0.5% by weight, preferably of not more than 0.4% by weight based on the weight of the crystalline (**1**), when heated from 30 to 180°C at a rate of 10 K/min; and/or
(x) a TGA curve that, when measured at a heating rate of 10 K/min, is essentially as shown in Figure 3; and/or
(xi) a combination of any two or more (i) - (x).

According to further aspect thereof, the present invention relates to a process for the preparation of crystalline rosuvastatin allyl ester (**1**), preferably crystalline form a, said process comprising:
a) contacting rosuvastatin allyl ester (**1**) in at least one liquid medium so as to obtain a mixture;
b) maintaining the mixture obtained in step a) under stirring so as to obtain a solution of rosuvastatin allyl ester (**1**) in the liquid medium;
c) optionally, when step b) has been performed to a temperature of or higher than 25 °C, cooling the solution obtained in step b) so as to cause precipitation of at least a portion of rosuvastatin allyl ester (**1**) in crystalline form; and
d) isolating and, optionally, drying it.

Step a) comprises contacting rosuvastatin allyl ester (**1**) with at least one liquid medium, preferably at temperatures from 5 °C to 30 °C, more preferably from 10 °C to 28 °C, even more preferably from 20 °C to 25 °C.

Suitable liquid mediums include nonpolar solvents, aprotic polar solvents, polar protic solvents, and combinations thereof. Non-limiting examples of suitable nonpolar solvents include toluene, *tert*-butyl methyl ether, di-*tert*-butyl ether, diethyl ether, diisopropyl ether, cyclopropyl methyl ether, and combinations thereof. Suitable aprotic solvents include, for example, acetonitrile, methyl acetate, ethyl acetate, isopropyl acetate, *n*-butyl acetate, isobutyl acetate, tetrahydrofuran, 2-methyl tetrahydrofuran, and combinations thereof. Polar protic solvents suitable for the aim include, e.g., alcohols such as methanol, ethanol, *n*-propanol, isopropanol, *n*-butanol, *iso*-butanol, *tert*-butanol, or mixtures thereof. In a preferred embodiment, the least one liquid medium is diethyl ether, ethyl acetate, methanol, ethanol or a mixture thereof. Even more preferably the least one liquid medium is diethyl ether.

The volume of the at least one liquid medium can vary in a very wide range; preferably, it is from 0.5 mL to 50 mL per gram of rosuvastatin allyl ester (**1**). Preferably the volume of the liquid medium is between and optionally includes any two of the following values: 0.6 mL, 0.7 mL, 0.8 mL, 0.9 mL, 1 mL, 2 mL, 3 mL, 4 mL, 5 m mL, 6 mL, 7 mL, 8 mL, 9 mL, 10 mL, 11 mL, 12 mL, 13 mL, 14 mL, 15 mL, 16 mL, 17 mL, 18 mL, 19 mL, 20 mL, 25 mL, 30 mL, 35 mL, 40 mL or 45 mL per gram of rosuvastatin allyl ester (**1**). More preferably said volume is from 5 mL to 15 mL per gram of rosuvastatin allyl ester (**1**).

Rosuvastatin allyl ester (**1**) suitable to be used in step a) is commercially available; alternatively, it can be prepared according to standard techniques in organic synthesis, e.g., according to the procedures described in WO 2011/160974 A1 or WO 2019/139919.

Preferably step a) is carried out by adding at least one liquid medium to a mass comprising, more preferably consisting of, rosuvastatin allyl ester (**1**).

The following step b) includes maintaining the mixture prepared in step a) under stirring so as to obtain a solution of rosuvastatin allyl ester (**1**) in the at least one liquid medium.

Preferably step b) is carried out at a temperature from 30 °C to the reflux temperature of the at least one liquid medium used, more preferably at a temperature from 32 °C to 100 °C, even more preferably from 35 °C to 80 °C.

Optional step c) includes, when step b) has been performed to a temperature of or higher than 25 °C, cooling the solution so as to cause precipitation of at least a portion of rosuvastatin allyl ester (1) in crystalline form, preferably the crystalline form a. This step is normally carried out by maintaining the solution prepared in step b) at temperatures from -20 °C to 20 °C (preferably from -10 °C to 15 °C, more preferably from 0 °C to 10 °C), preferably for at least 2 hours (more preferably for a period from 3 to 50 hours, even more preferably from 5 to 25 hours).

The cooling rate is thereby not critical and may range from about -0.1 °C/hour to -10 °C/hour, e.g., the cooling rate is from -0.5 °C/hour to -5 °C/hour. In one embodiment the solution is allowed to cool naturally to room temperature.

The crystalline form of rosuvastatin allyl ester (**1**), preferably crystalline form a, is recovered in step d) using known techniques such as filtration or centrifugation and optionally washed with a suitable liquid medium, preferably the same medium used in step a).

The obtained crystals may then optionally be dried. Drying may be performed at a temperature from 20 °C to 60°C, preferably from 25 °C to 40 °C. Drying may be performed for a period from about 1 to 72 hours, preferably from 2 to 48 hours, more preferably from 4 to 24 hours and most preferably from 6 to 18 hours. Drying may be performed at ambient pressure or under reduced pressure. Preferably, drying is performed at reduced pressure of about 100 mbar or less, more preferably of about 50 mbar or less, for example a *vacuum* of about 35 mbar is applied for drying. In a possible variant of this aspect of the invention, an optional step b') is carried out between steps b) and c), comprising seeding the solution prepared in step b) with rosuvastatin allyl ester (**1**), preferably crystalline form a, so as to promote precipitation of the same crystalline form. Preferably, the seed is added in a weight ratio from 0.5% to 10% (preferably from 1% to 5%) with respect to the amount of rosuvastatin allyl ester (**1**) used in step a) and at a temperature at least 10 °C lower than that used to operate step b).

By producing rosuvastatin allyl ester (**1**) and crystallizing it using the at least one liquid medium discussed above in connection with a) (preferably diethyl ether), the contents of the related impurities present in (**1**) (particularly its optical isomers, i.e. (3*R*,5*R*)-isomer and/or (3*S*,5*S*)-isomer and/or (3S,5R)-isomer) can be decreased, thus allowing for the preparation of crystalline rosuvastatin allyl ester (**1**), preferably of crystalline form a, in diasteromerically and/or enantiomerically pure form.

### Pharmaceutical compositions and medical use

In a further aspect, the present invention relates to the use of crystalline rosuvastatin allyl ester (**1**), preferably of crystalline form a, as defined in any one of the aspects and embodiments described above for the preparation of a pharmaceutical composition.

In a further aspect, the present invention relates to a pharmaceutical composition comprising crystalline rosuvastatin allyl ester (**1**), in particular crystalline form α of (**1**), as defined in any one of the aspects and embodiments described above, preferably in an effective amount, and optionally at least one pharmaceutically acceptable excipient.

Preferably, the effective amount of rosuvastatin allyl ester (**1**), in particular of the crystalline form α as defined in any one of the aspects and embodiments described above, is from 1 to 500 mg, for example, from 2.5 mg to 100 mg.

Pharmaceutically acceptable excipients suitable for the aim preferably include fillers (for example lactose, sucrose, glucose, mannitol, sorbitol, calcium phosphate, calcium carbonate, and cellulose), diluents, binders (e.g., gelatine, polyvinyl pyrrolidone, cellulose derivatives, polyethylene glycol, sucrose, and starch), disintegrants (for example starch, cellulose, crosslinked polyvinyl pyrrolidone, sodium starch glycolate and sodium carboxymethyl cellulose), lubricants (for example magnesium stearate, stearic acid, polyethylene glycol, sodium lauryl sulphate, sodium lauryl fumarate, and liquid paraffin), glidants (like silica, magnesium stearate, starch, cellulose, and talc) and combinations thereof.

Preferably, the at least one pharmaceutically acceptable excipient is selected from the group consisting of lactose (more preferably lactose monohydrate), microcrystalline cellulose, calcium phosphate, crospovidone, hypromellose, magnesium stearate and combinations thereof. Even more preferably, all of these pharmaceutically acceptable excipients are comprised by the pharmaceutical composition of the present invention.

Preferably, the pharmaceutical composition of the present invention as described above is an oral solid dosage form. More preferably, the pharmaceutical composition of the present invention as described above is a tablet or a capsule. In a particular embodiment, the pharmaceutical composition of the present invention as described above is a tablet, preferably a film-coated tablet.

In another particular embodiment, the pharmaceutical composition of the present invention as described above is a capsule, preferably a hard-gelatine capsule. In a further embodiment, the capsule shell is a gelatine shell or a hydroxypropyl methylcellulose (HPMC) shell, preferably a gelatine shell.

The pharmaceutical compositions of the present invention as defined in any one of the above-described embodiments may be produced by standard manufacturing processes, which are well-known to the skilled person including, e.g., blending, granulation (wet or dry granulation), tablet compression, film-coating or capsule filling.

The present invention also relates to a packaging comprising the pharmaceutical composition as defined in anyone of the embodiments described above, wherein the packaging is preferably a high-density polyethylene bottle.

In a further aspect, the present invention relates to crystalline rosuvastatin allyl ester (**1**), in particular crystalline form a, or the pharmaceutical composition comprising the same as defined in any one of the aspects and their corresponding embodiments described above for use as a medicament.

In a further aspect, the present invention relates to crystalline rosuvastatin allyl ester (**1**), in particular crystalline form a, or the pharmaceutical composition comprising the same, as defined in any one of the aspects and their corresponding embodiments described above, for use in medicine, in particular as a prodrug of a lipid-lowering agent, preferably rosuvastatin, for example in the treatment of homozygous familial hypercholesterolemia, hyperlipidaemia, mixed dyslipidaemia, primary dysbetalipoproteinemia, hypertriglyceridemia, and prevention of cardiovascular disease.

### EXAMPLES

The following non-limiting examples are illustrative for the disclosure and are not to be construed as to be in any way limiting for the scope of the invention.

The instruments and methods used to characterize the obtained solid form are as follows: **XRPD:** (XRD) patterns of the polymorphic form were recorded by a Empyrean diffractometer with a CuKa radiation. In order to focus only on indicative reflections assigned to the samples phase, a narrow 2Θ region from 2 to 40° was selected with a step of 0.026° per 50 s.

**DSC:** DSC tests were performed using Mettler Toledo DSC 3+ equipment (Mettler Toledo, Switzerland). The samples were heated from 30 to 300 °C at a rate of 10 K min⁻¹ in a nitrogen atmosphere. STARe software was used to determine transition temperatures and peak integrals. **TGA** measurements were performed on a TGA/DSC 1 thermogravimetric analyser (Mettled Toledo, Switzerland) at a heating rate of 10 K min⁻¹ from 30 to 300°C in a nitrogen atmosphere.

### Example 1: Preparation of rosuvastatin allyl ester (1)

To a solution of rosuvastatin calcium salt (10.0 g, 20.8 mmol) in DMF (100 mL) was added allyl bromide (10.1 g, 83.1 mmol). The solution was stirred at 25 °C and monitored by HPLC. After 20 hours, the reaction mixture was diluted with H₂O (200 mL) and extracted with ethyl acetate (3 x 200 mL). The combined organic layers were washed with brine (2 x 100 mL), dried over Na₂SO₄, filtered and concentrated under reduced pressure. The concentrate was purified by column chromatography (Biotage System, SNAP Cartridge silica 340 g, n-heptane : ethyl acetate =1:1 V/V) to give amorphous rosuvastatin allyl ester (**1**) (6.0 g, 57% yield) with chemical, enantiomeric and diastereomeric purity higher than 99.9%.

### Example 2: Preparation of crystalline form α of rosuvastatin allyl ester (1)

Amorphous rosuvastatin allyl ester (**1**) prepared according to example 1 (1.0 g) was dissolved in diethyl ether (10 mL) upon heating to reflux (about 35 °C). The resulting clear solution was cooled to about 5 °C in 15 hours then left upon stirring at the same temperature for another 5 hours. Finally, the obtained crystals were collected by filtration under *vacuum* (using a Büchner glass funnel with sintered disc) and subsequently dried under *vacuum* (at 40 °C and about 35 mbar) for 12 hours.

The obtained product was analysed by XRPD, obtaining the diffractogram shown in Figure 1. The product was also subjected to DSC and TGA analyses, which gave as results the traces shown in Figures 2 and 3, respectively.

## Claims

1. Crystalline rosuvastatin allyl ester (**1**)

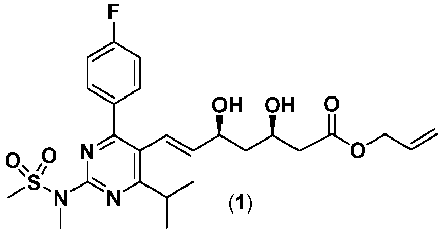

2. Crystalline form of rosuvastatin allyl ester (**1**) according to claim 1, **characterized in that** it is anhydrous and/or non-solvated and/or non-hygroscopic.

3. Crystalline form of rosuvastatin allyl ester (**1**) according to any one of claims 1 and 2, **characterized in that** it shows at least one of the following features (i) to (xi):
(i) an X-ray powder diffraction pattern that, when collected with the Kα radiation of copper (λ = 1.5419 Å) and a temperature from 20 to 30°C, shows main reflections at 16.14° (± 0.20° 2Θ) and 21.13° (± 0.20° 2Θ); and/or
(ii) an X-ray powder diffraction pattern that, when collected with the Kα radiation of copper (λ = 1.5419 Å) and a temperature from 20 to 30°C, shows main reflections at 7.70° (± 0.20° 2Θ), 16.14° (± 0.20° 2Θ), 19.53° (± 0.20° 2Θ) and 21.13° (± 0.20° 2Θ); and/or
(iii) an X-ray powder diffraction pattern that, when collected with the Kα radiation of copper (λ = 1.5419 Å) and a temperature from 20 to 30°C, shows main reflections at 7.70° (± 0.20° 2Θ), 8.41° (± 0.20° 2Θ), 14.99° (± 0.20° 2Θ), 16.14° (± 0.20° 2Θ), 19.53° (± 0.20° 2Θ), 21.13° (± 0.20° 2Θ) and 22.24° (± 0.20° 2Θ); and/or
(iv) an X-ray powder diffraction pattern that, when collected with the Kα radiation of copper (λ = 1.5419 Å) and a temperature from 20 to 30°C, shows main reflections at 7.70° (± 0.20° 2Θ), 8.41° (± 0.20° 2Θ), 14.99° (± 0.20° 2Θ), 16.14° (± 0.20° 2Θ), 16.87° (± 0.20° 2Θ), 18.44° (± 0.20° 2Θ), 19.53° (± 0.20° 2Θ), 21.13° (± 0.20° 2Θ), 22.24° (± 0.20° 2Θ) and 24.74° (± 0.20° 2Θ); and/or
(v) an X-ray powder diffraction pattern that, when collected with the Kα radiation of copper (λ = 1.5419 Å) and a temperature from 20 to 30°C, shows main reflections at 7.70° (± 0.20° 2Θ), 8.41° (± 0.20° 2Θ), 12.63° (± 0.20° 2Θ), 14.99° (± 0.20° 2Θ), 16.14° (± 0.20° 2Θ), 16.87° (± 0.20° 2Θ), 17.96° (± 0.20° 2Θ), 18.44° (± 0.20° 2Θ), 19.53° (± 0.20° 2Θ), 21.13° (± 0.20° 2Θ), 22.24° (± 0.20° 2Θ), 24.74° (± 0.20° 2Θ) and 28.55° (± 0.20° 2Θ); and/or
(vi) an X-ray powder diffraction pattern that, when collected with the Kα radiation of copper (λ = 1.5419 Å) and a temperature from 20 to 30°C, is essentially as shown in Figure 1; and/or
(vii) a DSC trace comprising an endothermic peak having an onset at a temperature of (93 ± 5)°C, preferably of (93 ± 3)°C, more preferably of (93 ± 2)°C, even more preferably of (93 ± 1)°C, when measured at a heating rate of 10 K/min; and/or
(viii) a DSC trace that, when measured at a heating rate of 10 K/min, is essentially as shown in Figure 2; and/or
(ix) a TGA curve showing a mass loss of not more than 0.5% by weight, preferably of not more than 0.4% by weight based on the weight of the crystalline (**1**), when heated from 30 to 180°C at a rate of 10 K/min; and/or
(x) a TGA curve that, when measured at a heating rate of 10 K/min, is essentially as shown in Figure 3; and/or
(xi) a combination of any two or more (i) - (x).

4. Use of crystalline rosuvastatin allyl ester (**1**) according to any one of claims 1 to 3 for the preparation of a pharmaceutical composition.

5. A pharmaceutical composition comprising, as active ingredient, crystalline rosuvastatin allyl ester (**1**) as defined in any one of claims 1 to 3 and at least one pharmaceutically acceptable excipient.

6. The composition according to claim 5 wherein the at least one pharmaceutically acceptable excipient is selected from the group consisting of fillers, diluents, binders, disintegrants, lubricants, glidants and combinations thereof.

7. Process for the preparation of a crystalline rosuvastatin allyl ester (**1**) according to any one of claims 1 to 3, said process comprising:
a) contacting rosuvastatin allyl ester (**1**) with at least one liquid medium so as to obtain a mixture;
b) maintaining the mixture obtained in step a) under stirring so as to obtain a solution of rosuvastatin allyl ester (**1**) in the liquid medium;
c) optionally, when step b) has been performed to a temperature of or higher than 25 °C, cooling the solution obtained in step b) so as to cause precipitation of at least a portion of rosuvastatin allyl ester (**1**) in crystalline form; and
d) isolating and, optionally, drying it.

8. Process according to claim 7 wherein, the at least one liquid medium used in step a) is selected from the group consisting of nonpolar solvents, aprotic polar solvents, polar protic solvents, and combinations thereof.

9. Process according to any one of claims 7 and 8 wherein, the at least one liquid medium used in step a) is diethyl ether.

10. Crystalline rosuvastatin allyl ester (**1**) as defined in any one of claims 1 to 3 or the pharmaceutical composition as defined in any one of claims 5 and 6 for use as a medicament.
